# EUROPÄISCHE PATENTSCHRIFT

(11) **EP 4 391 591 B1**
(45) Veröffentlichungstag und Bekanntmachung des Hinweises auf die Patenterteilung: **04.03.2026**
(21) Anmeldenummer: 23217134.8
(22) Anmeldetag: 15.12.2023
(51) Int. Cl.: H04R 25/00, A61B 5/12

(54) **MEHRKANALAUDIOMETER UND AUDIOMETRISCHES TESTSYSTEM**
MULTICHANNEL AUDIOMETER AND AUDIOMETRIC TEST SYSTEM
AUDIOMETRE MULTICANAL ET SYSTEME DE TEST AUDIOMETRIQUE

(30) Priorität: 21.12.2022 DE 202022107151 U
(43) Veröffentlichungstag der Anmeldung: 26.06.2024
(73) Patentinhaber: Auritec medizin-diagnostische Systeme GmbH, 22047 Hamburg (DE)
(72) Erfinder: MODY, Jan, 23758 Grammdorf (DE)
(74) Vertreter: Seemann & Partner Patentanwälte mbB

(56) Entgegenhaltungen:
- DE-A1- 10 212 765
- US-A- 5 197 332
- US-A1- 2012 067 107
- US-A1- 2021 274 296
- US-A1- 2023 121 895
- US-B2- 9 445 754

## Beschreibung

Die Erfindung betrifft ein Mehrkanalaudiometer, umfassend ein Grundmodul mit einem Tongenerator zum Erzeugen eines mehrkanaligen Testsignals, einem Pegelregler zur Veränderung eines Pegels des Testsignals und mit einer Schnittstelle, an der das mehrkanalige Testsignal bereitstellbar ist.

Audiometer sind diagnostische Geräte zur Bestimmung der menschlichen Hörfähigkeit. Für audiometrische Messungen sind verschiedene Systeme bekannt, mit denen die menschliche Hörfähigkeit gemessen wird, beispielsweise die Hörschwelle oder die Hörfähigkeit bei einzelnen Frequenzen. Bei einigen Audiometern wird über einen Kopfhörer ein Testsignal, beispielsweise ein gepulster Sinuston unterschiedlicher Frequenz oder genormtes Sprachmaterial, einem Testhörer zwecks Untersuchung bereitgestellt. Bei anderen Audiometern werden die Testsignale über Lautsprecher, d.h. in Freifeldübertragung, bereitgestellt. Bei einem solchen System entfällt zwar der vielfach als störend empfundene Kopfhörer, jedoch muss der Raum, in welchem die Messung durchgeführt wird, für diese eingerichtet sein, beispielsweise mit einer Schalldämmung versehen sein. Eine Vorrichtung sowie ein Verfahren der Schallfeldaudiometrie sind beispielsweise aus der DE 102 12 765 A1 bekannt.

US 5,197,332 A1 zeigt eine Kopfhörer-basierte Vorrichtung zum Testen und Verbessern der Hörfähigkeit.

In US 9,445,754 B2 ist ein Verfahren und ein System gezeigt, die zum Anpassen von Hörgeräten, zum Trainieren der Nutzung von Hörgeräten und zum Durchführen von Tests mit Personen, die Hörgeräte tragen, vorgesehen sind.

US 2021/0274296 A1 zeigt ein Hörhilfensystem zum Abschätzen von akustischen Transferfunktionen.

Es ist eine Aufgabe der Erfindung ein Mehrkanalaudiometer sowie ein audiometrisches Testsystem anzugeben, welches einen erweiterten Anwendungsbereich aufweist.

Die Aufgabe wird gelöst durch ein Mehrkanalaudiometer, umfassend ein Grundmodul mit einem Tongenerator zum Erzeugen eines mehrkanaligen Testsignals, einem Pegelregler zur Veränderung eines Pegels des Testsignals und mit einer Schnittstelle, an der das mehrkanalige Testsignal bereitstellbar ist, wobei das Audiometer fortgebildet ist durch eine mit der Schnittstelle gekoppelte Sendeeinheit, die dazu eingerichtet ist das Testsignal über eine drahtlose Datenverbindung mit kurzer Reichweite, insbesondere über eine Bluetooth-Datenverbindung, bereitzustellen, wobei die Sendeeinheit dazu eingerichtet ist, das Testsignal über die drahtlose Datenverbindung mit kurzer Reichweite an einen Sprachprozessor eines Hörimplantats zu übertragen, wobei es sich bei der drahtlosen Datenverbindung um eine bidirektionale Datenverbindung handelt, wobei die Sendeeinheit und die Empfangseinheit jeweils als Sende-/Empfangseinheiten ausgebildet sind, wobei das Grundmodul mehrere mit der Schnittstelle gekoppelte Audioausgänge umfasst, an denen das mehrkanalige Testsignal als ein für eine Freifeldübertragung ausgelegtes mehrkanaliges Ausgangssignal bereitstellbar ist.

Das Testsignal umfasst mehrere Kanäle, z.B. einen rechten und einen linken Kanal. Es kann sich bei dem Testsignal ebenfalls um ein Signal handeln, welches mehr als zwei Kanäle aufweist und beispielsweise einem Surround System zugeführt werden kann.

Vorteilhaft ist mit dem Audiometer eine Ankopplung eines Audiogeräts möglich, welches über eine entsprechende Empfangseinheit zum Betrieb der drahtlosen Datenverbindung, verfügt. Dies erweitert den Anwendungsbereich des Mehrkanalaudiometers. Beispielsweise sind viele Hörgeräte dazu eingerichtet, Audiosignale über eine drahtlose Datenverbindung, insbesondere über eine Bluetooth-Datenverbindung, zu empfangen. Testhörer, welche solche Hörgeräte tragen, können zum Zweck eines Hörtests ihre Hörgeräte direkt mit dem Audiometer koppeln, sodass anschließend eine Messung durchgeführt werden kann.

Insbesondere ist das Mehrkanalaudiometer dadurch fortgebildet, dass die Sendeeinheit dazu eingerichtet ist die drahtlose Datenverbindung, insbesondere die Bluetooth-Datenverbindung, auf der Grundlage von 2,4-GHz-Übertragungstechnik zu betreiben. Die Verwendung dieser Übertragungstechnik ermöglicht es dem Audiometer beispielsweise eine Bluetooth-Datenverbindung zu einem Hörgerät aufzubauen. Medizinische Geräte, welche Bluetooth-Datenverbindungen betreiben, kommunizieren vielfach in dem genannten Frequenzbereich.

Gemäß einer Ausführungsform umfasst das Mehrkanalaudiometer einen Sprachprozessor eines solchen Hörgeräts, beispielsweise eine Hörimplantats, welches über eine Bluetooth Konnektivität verfügt. Der Sprachprozessor des Hörgeräts umfasst eine entsprechend eingerichtete Empfangseinheit. Die Sendeeinheit des Grundmoduls und die Empfangseinheit des Hörgeräts sind dazu eingerichtet die Bluetooth-Datenverbindung zu betreiben. Insbesondere ist die Sendeeinheit des Grundmoduls dazu eingerichtet das Testsignal über die Bluetooth-Datenverbindung an die Empfangseinheit des Hörgeräts zu übertragen.

Das Grundmodul umfasst mehrere mit der Schnittstelle gekoppelte Audioausgänge, an denen das mehrkanalige Testsignal als ein für eine Freifeldübertragung ausgelegtes mehrkanaliges Ausgangssignal bereitstellbar ist. Das Mehrkanalaudiometer umfasst ferner insbesondere mit den Audioausgängen gekoppelte Lautsprecher. Ausgehend von diesen Lautsprechern kann die Freifeldübertragung bis zu einem Testplatz erfolgen, an dem sich ein Testhörer befindet, dessen Hörfähigkeit mit Hilfe des Audiometers untersuchbar ist. Vorteilhaft ist es mit dem Mehrkanalaudiometer möglich, das Testsignal sowohl über die drahtlose Datenverbindung als auch per akustischer Freifeldübertragung an den Testhörer zu kommunizieren. Für beide Fälle kann die Hörfähigkeit eines Testhörers miteinander verglichen werden. Diese Information kann beispielsweise zur Einstellung des Hörgeräts hilfreich sein.

Gemäß einer weiteren Ausführungsform ist das Mehrkanalaudiometer dadurch fortgebildet, dass der Pegelregler dazu eingerichtet ist, auf der Grundlage der Daten einer Kalibrierungsmessung einen Pegel des über die drahtlose Datenverbindung, insbesondere über die Bluetooth-Datenverbindung, bereitstellbaren Testsignals und einen Pegel des für die Freifeldübertragung ausgelegten Ausgangssignals aufeinander abzustimmen.

Durch die Kalibrierungsmessung wird sichergestellt, dass ein Signal für den Testhörer immer die gleiche Lautstärke hat, gleichgültig ob er es über die Freifeldübertragung, beispielsweise mittels der Lautsprecher, empfängt oder ob dieses über die drahtlose Datenverbindung übertragen wird. Das Mehrkanalaudiometer bietet somit die Möglichkeit eines quantitativen Vergleichs dieser beiden Hörwege.

Das Mehrkanalaudiometer ist dadurch fortgebildet, dass die Sendeeinheit dazu eingerichtet ist, das Testsignal über die drahtlose Datenverbindung mit kurzer Reichweite, insbesondere über eine Bluetooth-Datenverbindung, an einen Sprachprozessor eines Hörimplantats, insbesondere eines Cochlea-Implantats oder eines Mittelohrimplantats, zu übertragen.

Ein Hörimplantat, beispielsweise ein Cochlea-Implantat oder ein Mittelohrimplantat, umfasst das eigentliche Implantat, dessen Elektrode beispielsweise in die Cochlea eines Patienten eingesetzt ist. Das Implantat umfasst ferner eine patientenseitige Empfangsspule, welche Signale empfängt, die über die Elektrode in die Cochlea des Patienten weitergeleitet werden. Die Empfangsspule des Implantats koppelt mit einer Sendespule eines Sprachprozessors, welcher beispielsweise hinter dem Ohr getragen wird. Über die Sende- und Empfangsspulen werden die zur Erzeugung des Höreindrucks erforderlichen Signale übermittelt. Die entsprechenden Signale erzeugt der Sprachprozessor des Implantats. Der Sprachprozessor umfasst ein Mikrofon zum Empfang von akustischen Signalen. Der Sprachprozessor stellt in vielen Fällen auch eine Konnektivität für eine drahtlose Datenverbindung, beispielsweise eine Bluetooth-Empfangseinheit, zur Verfügung. Auf dieser Grundlage ist es möglich, eine direkte Übertragung des Testsignals von dem Grundmodul des Mehrkanalaudiometers zu dem Sprachprozessor beispielsweise eines Cochlea-Implantats vorzunehmen. In Verbindung mit einer Kalibrierungsmessung kann eine Kontrolle auf dem Grundsatz der Äquivalenz der Übertragungswege vorgenommen werden, was beispielsweise eine hochgenaue Kalibrierung des Cochlea-Implantats ermöglicht. Die vorgenannten Ausführungsformen gelten selbstverständlich in gleicher Weise für ein Mittelohrimplantat.

Die Aufgabe wird ferner gelöst durch ein audiometrisches Testsystem, umfassend ein Mehrkanalaudiometer nach einer oder mehreren der zuvor genannten Ausführungsformen sowie einen Sprachprozessor eines Hörimplantats, insbesondere eines Cochlea-Implantats oder eines Mittelohrimplantats, wobei der Sprachprozessor eine weitere Empfangseinheit umfasst, und wobei die Sendeeinheit des Grundmoduls des Mehrkanalaudiometers dazu eingerichtet ist das Testsignal über die drahtlose Datenverbindung, insbesondere die Bluetooth-Datenverbindung, an die Empfangseinheit des Sprachprozessors des Hörimplantats zu übertragen.

Auf das audiometrische Testsystem treffen gleiche oder ähnliche Vorteile zu wie sie bereits zuvor im Hinblick auf das Mehrkanalaudiometer erwähnt wurden. Zur Vermeidung von Wiederholungen sollen diese nicht erneut genannt werden.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematisch vereinfachte Darstellung eines Mehrkanalaudiometers und
- Fig. 2: Vergleichskurven der Übertragungspegel einer Freifeldübertragung und einer Übertragung des Testsignals über die Bluetooth-Datenverbindung.

In den Zeichnungen sind jeweils gleiche oder gleichartige Elemente und/oder Teile mit denselben Bezugsziffern versehen, so dass von einer erneuten Vorstellung jeweils abgesehen wird.

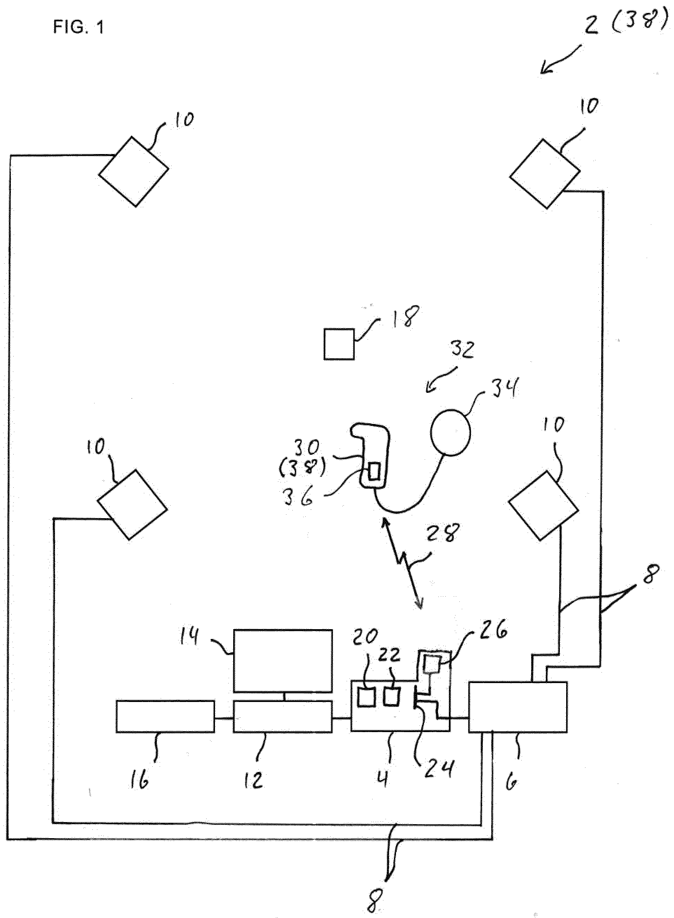

Fig. 1 zeigt in schematisch vereinfachter Darstellung ein Mehrkanalaudiometer 2 in einer ebenfalls schematisch dargestellten Messumgebung. Das Mehrkanalaudiometer 2 umfasst ein Grundmodul 4, ein Surround Modul 6, welches über mehrere Verbindungsleitungen 8 mit beispielhaft vier Lautsprechern 10 verbunden ist, einen Steuerungsrechner 12 mit einem Bildschirm 14 sowie ein Bedienteil 16. Das Surround Modul 6 kann in das Grundmodul 4 integriert sein. Die Lautsprecher 10 sind auf einen Messplatz 18 ausgerichtet, auf dem ein Testhörer Platz nehmen kann. Am Messplatz 16 kann mit Hilfe des Mehrkanalaudiometers 2 eine Messung der Hörfähigkeit eines Testhörers durchgeführt werden. Bei dem Steuerungsrechner 12 handelt es sich beispielsweise um einen speziell eingerichteten Silent-PC mit geringem Betriebsgeräusch. Das Surround Modul 6 bietet im dargestellten Ausführungsbeispiel vier Freifeldausgänge an denen die Verbindungsleitungen 8, welche zu den Lautsprechern 10 führen, angeschlossen sind. Das Surround Modul 6 kann mehr als vier Freifeldausgänge, beispielsweise acht oder sechzehn Freifeldausgänge, umfassen. So erhält der Nutzer die Möglichkeit eigene Messkonfigurationen anzulegen, auch solche mit hoher Komplexität, und gleichzeitig mehrere unterschiedliche Kanäle wiederzugeben, an die unterschiedliche Signale angelegt werden können. Dies kann in einer entsprechenden Benutzeroberfläche einer Steuerungssoftware, die auf dem Steuerungsrechner 12 läuft, dargestellt werden. Das Mehrkanalaudiometer 2 ist sowohl für die Sprachaudiometrie als auch für die Tonaudiometrie eingerichtet und geeignet. Es bietet die Möglichkeit, für jeden einzelnen Kanal Signale auszuwählen, Richtungs- und Pegeleinstellungen vorzunehmen und dies gleichzeitig zu visualisieren.

Das Grundmodul 4 umfasst zum Erzeugen eines mehrkanaligen Testsignals, bei dem es sich beispielsweise um ein Tonsignal oder um ein Sprachsignal handelt, einen Tongenerator 20. Ferner umfasst das Grundmodul 4 einen Pegelregler 22 zur Veränderung eines Pegels des Testsignals. Das Testsignal wird an einer Schnittstelle 24 des Grundmoduls 4 bereitgestellt. Ausgehend von der Schnittstelle 24 gelangt das Testsignal beispielsweise zu dem Surround Modul 6 und von dort weiter zur Lautsprecherausgabe. Das Grundmodul 4 umfasst ferner eine Sendeeinheit 26, die ebenfalls mit der Schnittstelle 24 gekoppelt und dazu eingerichtet ist, das Testsignal über eine drahtlose Datenverbindung 28 mit kurzer Reichweite, beispielsweise eine Bluetooth-Datenverbindung, bereitzustellen.

Die Sendeeinheit 26 des Grundmoduls 4 ist insbesondere dazu eingerichtet, die drahtlose Datenverbindung 28 auf der Grundlage von 2,4-GHz-Übertragungstechnik zu betreiben. Dies macht die drahtlose Datenverbindung 28 besonders geeignet für die Kommunikation mit medizinischen Geräten, welche dazu eingerichtet sind, Datenverbindungen auf der Grundlage von Bluetooth herzustellen.

Das Grundmodul 4 kann ferner dazu eingerichtet sein, dass mit der Schnittstelle 24 mehrere Audioausgänge gekoppelt sind. Im dargestellten Beispiel sind diese Audioausgänge beispielhaft durch das Surround Modul 6 bereitgestellt. Auch über diese Audioausgänge, die direkt an dem Grundmodul 4 vorhanden sein können, kann das Testsignal, beispielsweise für eine Freifeldübertragung, bereitgestellt werden.

Der Pegelregler 22 des Grundmoduls 4 ist insbesondere dazu eingerichtet, auf der Grundlage der Daten einer Kalibriermessung einen Pegel des über die drahtlose Datenverbindung 28 bereitgestellten Testsignals und einen Pegel des für die Freifeldübertragung ausgelegten Ausgangssignals aufeinander abzustimmen. Eine entsprechende Kalibriermessung der Pegel wird am Messplatz 18 vorgenommen.

Insbesondere ist das Mehrkanalaudiometer 2 derart ausgelegt, dass dessen Sendeeinheit 26 dazu eingerichtet ist, das Testsignal über die Datenverbindung 28 an einen Sprachprozessor 30 eines Hörimplantats 32 zu übertragen. In Fig. 1 ist als beispielhaftes Hörimplantat 32 schematisch und nicht in maßstabsgetreuer Darstellung ein Cochlea-Implantat gezeigt. Dieses umfasst neben dem Sprachprozessor 30 eine Sendespule 34, welche mit einer patientenseitigen Empfangsspule zusammenwirkt. Der Sprachprozessor 30 umfasst eine Empfangseinheit 36, die dazu eingerichtet ist, mit der Sendeeinheit 26 des Grundmoduls 4 die drahtlose Datenverbindung 28 zu betreiben. Bei der drahtlosen Datenverbindung 28 handelt es sich um eine bidirektionale Datenverbindung. Bei dei bidirektionalen Datenverbindung werden zusätzlich zu den Daten des Testsignals beispielsweise Steuerdaten, beispielsweise während eines Kopplungsvorgangs der Bluetooth-Datenverbindung, zwischen der Sendeeinheit 26 und der Empfangseinheit 36 ausgetauscht. Bei der bidirektionalen Datenverbindung ist sowohl die Sendeeinheit 26 als auch die Empfangseinheit 36 als Sende-/Empfangseinheit ausgebildet.

Der Sprachprozessor 30 des Hörimplantats 32 kann gemeinsam mit dem Mehrkanalaudiometer 2 ein audiometrisches Testsystem 38 ausbilden. In diesem audiometrischen Testsystem 38 wird das von dem Grundmodul 4 des Mehrkanalaudiometers 2 erzeugte Testsignal über dessen Sendeeinheit 26 und die drahtlose Datenverbindung 28 an die Empfangseinheit 36 des Sprachprozessors 30 übertragen.

Die Sendeeinheit 26 kann abweichend von der Darstellung in Fig. 1 als separate Einheit ausgebildet sein. In einem solchen Fall umfasst das Grundmodul 4 einen entsprechenden Ausgang, an den die Sendeeinheit 26 angeschlossen wird.

Die direkte Ansteuerung von Hörimplantaten 32 unter Nutzung eines besonderen Hardwareausgangs des Grundmoduls 4 hat verschiedene Vorteile. Zum einen ergibt sich eine Reduktion von Störeinflüssen aus der Raumakustik oder sonstigen Quellen oder der komplette Verzicht auf Vertäubung bei monauralen oder dichotischen Messungen. Die Ansteuerung des Hörimplantats 32 kann alternativ zu der Datenübertragung über die drahtlose Datenverbindung 28 auch direkt über ein Kabel erfolgen. Dieses Übertragungskabel wird mit einem an dem Grundmodul 4 vorhandenen Ausgang auf der einen Seite und mit entweder dem Sprachprozessor 30 oder direkt mit dem patientenseitig vorhandenen Implantatteil verbunden. Bevorzugt ist jedoch die drahtlose Datenübertragung.

Mit Hilfe der bereits zuvor erwähnten Kalibriermessung wird sichergestellt, dass ein Signal für den Patienten immer die gleiche Lautstärke hat, gleichgültig ob es über die Lautsprecher 10, also im Freifeld, abgespielt oder über den speziellen Implantat Ausgang des Audiometers 2 übertragen wird.

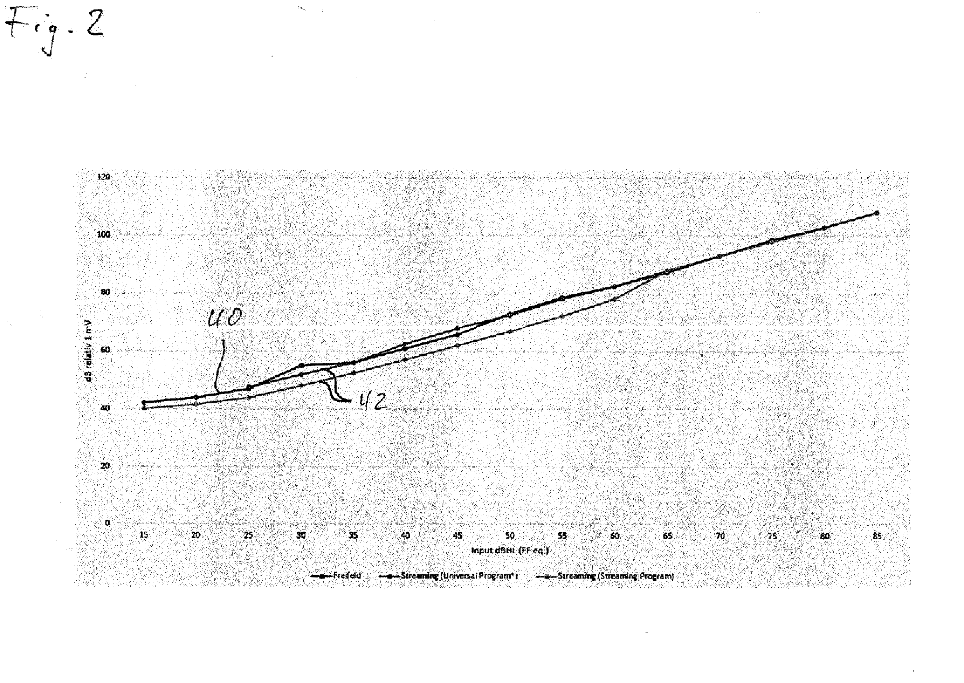

In Fig. 2 ist das Ergebnis einer Kalibrierungskontrolle gezeigt. Während die mit Bezugszeichen 40 bezeichnete Kurve die Freifeldübertragung kennzeichnet, zeigen die mit 42 bezeichneten Kurven die Übertragung mittels Audiolink, also einem Kabel, bzw. mittels der drahtlosen Datenverbindung 28, insbesondere Bluetooth. Es kann eine deutliche Übereinstimmung der Übertragungskurven festgestellt werden, was den Grundsatz der Äquivalenz der Übertragungswege bestätigt.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Die Erfindung wird durch die beigefügten Ansprüche definiert.

### Bezugszeichenliste

- 2: Mehrkanalaudiometer
- 4: Grundmodul
- 6: Surround Modul
- 8: Verbindungsleitungen
- 10: Lautsprecher
- 12: Steuerungsrechner
- 14: Bildschirm
- 16: Bedienteil
- 18: Messplatz
- 20: Tongenerator
- 22: Pegelregler
- 24: Schnittstelle
- 26: Sendeeinheit
- 28: drahtlose Datenverbindung
- 30: Sprachprozessor
- 32: Hörimplantat
- 34: Sendespule
- 36: Empfangseinheit
- 38: audiometrisches Testsystem
- 40: Freifeldübertragung
- 42: drahtlose Übertragung

## Patentansprüche

1. Mehrkanalaudiometer (2), umfassend ein Grundmodul (4) mit einem Tongenerator (20) zum Erzeugen eines mehrkanaligen Testsignals, einem Pegelregler (22) zur Veränderung eines Pegels des Testsignals und mit einer Schnittstelle (24), an der das mehrkanalige Testsignal bereitstellbar ist, **gekennzeichnet durch** eine mit der Schnittstelle (24) gekoppelte Sendeeinheit (26), die dazu eingerichtet ist das Testsignal über eine drahtlose Datenverbindung (28) mit kurzer Reichweite bereitzustellen, wobei die Sendeeinheit (26) dazu eingerichtet ist, das Testsignal über die drahtlose Datenverbindung (28) mit kurzer Reichweite an einen Sprachprozessor (30) eines Hörimplantats (32) zu übertragen, wobei es sich bei der drahtlosen Datenverbindung (28) um eine bidirektionale Datenverbindung handelt, wobei die Sendeeinheit (26) und die Empfangseinheit (36) jeweils als Sende-/Empfangseinheiten ausgebildet sind, **dadurch** gekennzeichnet, dass das Grundmodul (4) mehrere mit der Schnittstelle (24) gekoppelte Audioausgänge umfasst, an denen das mehrkanalige Testsignal als ein für eine Freifeldübertragung ausgelegtes mehrkanaliges Ausgangssignal bereitstellbar ist.

2. Mehrkanalaudiometer (2) nach Anspruch 1, bei dem die Sendeeinheit (26) dazu eingerichtet ist die drahtlose Datenverbindung (26) mit kurzer Reichweite auf der Grundlage von 2,4-GHz-Übertragungstechnik zu betreiben.

3. Mehrkanalaudiometer (2) nach Anspruch 1 oder 2, bei dem der Pegelregler (22) dazu eingerichtet ist, auf der Grundlage der Daten einer Kalibrierungsmessung einen Pegel des über die drahtlose Datenverbindung (28) bereitstellbaren Testsignals und einen Pegel des für die Freifeldübertragung ausgelegten Ausgangssignals aufeinander abzustimmen.

4. Mehrkanalaudiometer (2) nach einem der vorhergehenden Ansprüche, bei dem die Sendeeinheit (26) dazu eingerichtet ist das Testsignal über die drahtlose Datenverbindung (28) mit kurzer Reichweite an einen Sprachprozessor (30) eines Cochlea-Implantats oder eines Mittelohrimplantats, zu übertragen.

5. Mehrkanalaudiometer (2) nach einem der vorhergehenden Ansprüche, bei dem die Sendeeinheit (26) dazu eingerichtet ist, das Testsignal über eine Bluetooth-Datenverbindung an den Sprachprozessor (30) des Hörimplantats (32) zu übertragen.

6. Audiometrisches Testsystem (38) umfassend ein Mehrkanalaudiometer (2) nach einem der vorhergehenden Ansprüche und einen Sprachprozessor (30) eines Hörimplantats (32), insbesondere eines Cochlea-Implantats oder eines Mittelohrimplantats, wobei der Sprachprozessor (30) eine weitere Empfangseinheit (36) umfasst, und wobei die Sendeeinheit (26) des Grundmoduls (4) des Mehrkanalaudiometers (2) dazu eingerichtet ist das Testsignal über die drahtlose Datenverbindung (28), insbesondere eine Bluetooth-Datenverbindung, an die Empfangseinheit (36) des Sprachprozessors (30) des Hörimplantats (32) zu übertragen.

## Claims

1. A multi-channel audiometer (2), comprising a base module (4) having a tone generator (20) for generating a multi-channel test signal, a level controller (22) for changing a level of the test signal and having an interface (24) at which the multi-channel test signal can be provided, **characterized by** a transmission unit (26) that is coupled to the interface (24) and that is configured to provide the test signal via a wireless data connection (28) with a short range, wherein the transmission unit (26) is configured to transmit the test signal via the wireless data connection (28) with a short range to a speech processor (30) of a hearing implant (32), wherein the wireless data connection (28) is a bidirectional data connection, wherein the transmission unit (26) and the reception unit (36) are each designed as transceiver units, **characterized in that** the base module (4) comprises multiple audio outputs which are coupled to the interface (24) and at which the multi-channel test signal can be provided as a multi-channel output signal that is designed for free-field transmission.

2. The multi-channel audiometer (2) according to claim 1, wherein the transmission unit (26) is configured to operate the wireless data connection (26) with a short range on the basis of 2.4 GHz transmission technology.

3. The multi-channel audiometer (2) according to claim 1 or 2, wherein the level controller (22) is configured to coordinate a level of the test signal that can be provided via the wireless data connection (28) and a level of the output signal designed for free-field transmission with one another on the basis of the data of a calibration measurement.

4. The multi-channel audiometer (2) according to any one of the preceding claims, wherein the transmission unit (26) is configured to transmit the test signal via the wireless data connection (28) with a short range to a speech processor (30) of a cochlear implant or middle ear implant.

5. The multi-channel audiometer (2) according to any one of the preceding claims, wherein the transmission unit (26) is configured to transmit the test signal via a Bluetooth data connection to the speech processor (30) of the hearing implant (32).

6. An audiometric test system (38) comprising a multi-channel audiometer (2) according to any one of the preceding claims and a speech processor (30) of a hearing implant (32), in particular of a cochlear implant or middle ear implant, wherein the speech processor (30) comprises a further reception unit (36), and wherein the transmission unit (26) of the base module (4) of the multi-channel audiometer (2) is configured to transmit the test signal via the wireless data connection (28), in particular a Bluetooth data connection, to the reception unit (36) of the speech processor (30) of the hearing implant (32).

## Revendications

1. Audiomètre multicanal (2), comprenant un module de base (4) avec un générateur de sons (20) pour générer un signal de test multicanal, un régulateur de niveau (22) pour modifier le niveau du signal de test, et avec une interface (24) à laquelle le signal de test multicanal est apte à être fourni, **caractérisé par** une unité d'émission (26) reliée à l'interface (24), qui est conçue pour fournir le signal de test via une connexion de données sans fil (28) à courte portée, l'unité d'émission (26) étant conçue pour transmettre le signal de test à un processeur vocal (30) d'un implant auditif (32) via la connexion de données sans fil (28) à courte portée, la connexion de données sans fil (28) étant une connexion de données bidirectionnelle, l'unité d'émission (26) et l'unité de réception (36) étant chacune conçues sous la forme d'unités d'émission/réception, **caractérisé en ce que** le module de base (4) comprend plusieurs sorties audio reliées à l'interface (24), auxquelles le signal de test multicanal est apte à être fourni sous la forme d'un signal de sortie multicanal conçu pour une transmission en champ libre.

2. Audiomètre multicanal (2) selon la revendication 1, dans lequel l'unité d'émission (26) est conçue pour exploiter la connexion de données sans fil (26) à courte portée sur la base d'une technologie de transmission à 2,4 GHz.

3. Audiomètre multicanal (2) selon la revendication 1 ou la revendication 2, dans lequel le régulateur de niveau (22) est conçu pour ajuster, sur la base des données d'une mesure d'étalonnage, un niveau du signal de test apte à être fourni via la connexion de données sans fil (28) et un niveau du signal de sortie conçu pour une transmission en champ libre.

4. Audiomètre multicanal (2) selon l'une des revendications précédentes, dans lequel l'unité d'émission (26) est conçue pour transmettre le signal de test via la connexion de données sans fil (28) à courte portée à un processeur vocal (30) d'un implant cochléaire ou d'un implant de l'oreille moyenne.

5. Audiomètre multicanal (2) selon l'une des revendications précédentes, dans lequel l'unité d'émission (26) est conçue pour transmettre le signal de test au processeur vocal (30) de l'implant auditif (32) via une connexion de données Bluetooth.

6. Système de test audiométrique (38), comprenant un audiomètre multicanal (2) selon l'une des revendications précédentes et un processeur vocal (30) d'un implant auditif (32), en particulier d'un implant cochléaire ou d'un implant de l'oreille moyenne, le processeur vocal (30) comprenant une autre unité de réception (36), et l'unité d'émission (26) du module de base (4) de l'audiomètre multicanal (2) étant conçue pour transmettre à l'unité de réception (36) du processeur vocal (30) de l'implant auditif (32) le signal de test via la connexion de données sans fil (28), en particulier une connexion de données Bluetooth.
